# EUROPÄISCHE PATENTSCHRIFT

(11) **EP 0 513 042 B1**
(45) Veröffentlichungstag und Bekanntmachung des Hinweises auf die Patenterteilung: **05.01.1994**
(21) Anmeldenummer: 91902424.0
(22) Anmeldetag: 21.01.1991
(51) Int. Cl.: A61F 5/56

(54) **VORRICHTUNG ZUM REDUZIEREN DES SCHNARCHENS**
DEVICE FOR REDUCING SNORING
DISPOSITIF DESTINE A REDUIRE LE RONFLEMENT D'UN DORMEUR

(30) Priorität: 24.01.1990 DE 4001991
(43) Veröffentlichungstag der Anmeldung: 19.11.1992
(73) Patentinhaber: THOMSEN, Lorenz, D-21244 Buchholz (DE)
(72) Erfinder: THOMSEN, Lorenz, D-21244 Buchholz (DE)
(74) Vertreter: Schupfner, Gerhard D.
(86) Internationale Anmeldenummer: DE9100047
(87) Internationale Veröffentlichungsnummer: WO9111157

(56) Entgegenhaltungen:
- EP-A- 0 286 248
- DE-A- 3 300 851
- GB-A- 2 208 003

## Beschreibung

Die Erfindung bezieht sich auf eine Vorrichtung zum Reduzieren des Schnarchens eines Schläfers, dessen Kopf während des Schlafens auf einem Kissen ruht, das ein Abrollgebilde aufweist, das sich im Kissenbereich in Verlängerung der Längsmittellinie des Körpers des Schläfers erstreckt u. diesen zum seitlichen Abrollen zuingt.

Das Schnarchgeräusch entsteht während des Schlafens u. a. dadurch, daß bei offenem Mund in überwiegender Rückenlage das erschlaffte Gaumensegel in Schwingungen gerät. Zum einen kann diese Geräuschbelästigung den Schlafpartner erheblich in seiner Nachtruhe und damit gesundheitlich beeinträchtigen. Zum anderen bewirkt die offene Mundhaltung ein Austrocknen der Schleimhäute im Rachen und damit eine Gesundheitsgefährdung durch Neigung zu Erkältungen und Entzündungen. Darüber hinaus kann es beim Schnarchen zum Atemstillstand und damit zur Verminderung der Gehirndurchblutung mit entsprechenden Folgen kommen.

Aus der Europäischen Patentanmeldung 286 248 ist ein Kissen bekannt, das dem Reduzieren des Schnarchens dienen soll. Dieses Kissen ist mit zwei in entgegengesetzten Richtungen in das Kissenmaterial hineingeführten Kanälen versehen, die miteinander und mit der Längsmittellinie des Schläfers fluchten. In die beiden Kanäle ist ein relativ harter, stabförmiger Gegenstand eingesteckt, der zwischen den Kanälen freiliegend bleibt. Legt sich ein Schläfer auf dieses Kissen, dann stört der relativ harte Stab den Schläfer derart, daß er seinen Kopf zur Seite rollt.

Ein relativ harter Stab, der beispielsweise aus Gummi bestehen soll und eine im wesentlichen zylindrische Gestalt haben kann, ist außerordentlich unkomfortabel für den Schläfer, da selbst der zur Seite gerollte Kopf mit der Schädelhinterseite immer noch gegen den harten, gerade in Schädelmitte freiliegenden Stab stößt.

Es ist Aufgabe der Erfindung, eine Vorrichtung zu schaffen, bei der der Kopf des Schläfers auf komfortable, jedoch auch einen deutlichen Zwang ausübende Weise zu einem seitlichen Abrollen veranlaßt wird, mit der zugleich die Mundpartie vermehrt geschlossen gehalten wird und mit der evtl. Schnarchgeräusche gedämpft werden.

Die gestellte Aufgabe ist erfindungsgemäß dadurch gelöst, daß das wulstartig ausgebildete Abrollgebilde vom seinem dem Körper des Schläfers zugewandten Ende her und zum oben gelegenen Randbereich hin abgerundet augebildet ist und aus einem auffedernd nachgiebigen Material besteht.

Ein derartiges Abrollgebilde übt von seinem nackenseitigen Längsende her einen zunehmenden Abrollzwang aus, weil mit zunehmender Breite die Auffederungskraft des auffedernd nachgiebigen Materials zunimmt und diese Auffederungskraft den Kopf damit mit sanftem Druck zur Seite drängt. Der Schläfer berührt nach dem Abrollen mit dem Hinterkopf einen relativ weichen Gegenstand, der im Schlaf nicht stört, der das Zur-Seite-legen jedoch auch im Schlaf unbewußt aufrechterhält. Der Körper des Schläfers folgt dem Zurseitelegen des Kopfes unbewußt und legt sich auf zur Seite.

Nach einer weiteren Ausgestaltung der Erfindung ist vorgesehen, daß das Abrollgebilde aus einem geformten Kunststoffschaum oder Gummiwerkstoff, wie flexiblem Latexgummi besteht. Ein solches Abrollgebilde läßt sich leicht serienmäßig herstellen und in der Nachgiebigkeit und den Auffederungseigenschaften den Wünschen anpassen.

Bei einer abgewandelten Ausführungsform der Erfindung ist vorgesehen, daß das Abrollgebilde ein luftgefüllter Aufblaskörper ist. Ein solches Abrollgebilde braucht nur bei Bedarf aufgewölbt zu sein und ist unaufgeblasen nicht zu bemerken.

Nach einer weiteren Ausgestaltung der Erfindung ist vorgesehen, daß das Abrollgebilde von beiden Längsenden her zur Mitte hin rund oder oval bauchig breiter werdend ausgebildet ist. Dieses Abrollgebilde kann mit seinem ovalen Querschnitt sowohl liegend als auch stehend eingesetzt werden.

Nach einer weiteren Ausgestaltung der Erfindung ist vorgesehen, daß das Abrollgebilde an dem, seinem oben gelegenen Bereich gegenüberliegenden Randbereich mit einer fußförmigen Verbreiterung versehen ist. Ein solches Abrollgebilde kommt insbesondere dann in Betracht, wenn ein Kissen eine sehr weiche Kissenfüllung, beispielsweise aus Federn oder Daunen aufweist. Dann hat das Abrollgebilde im Kissen oder auf der Matratze eine besser verrollsichere Auflage.

Nach einer weiteren Ausgestaltung der Erfindung ist vorgesehen, daß das Abrollgebilde in oder an dem Formkissen angeordnet ist. Dieses Anordnen kann durch Ein- oder Aufkleben oder Befestigen mittels eines übergelegten Vlieses erfolgen.

Nach einer weiteren vorteilhaften Ausgestaltung der Erfindung ist vorgesehen, daß das Abrollgebilde auswechselbar an einem Kissenbezug angeordnet ist. In diesem Fall können handelsübliche Kisseninnenteile aus Federn, Kunststoffüllungen, Wolle, Schaumstoffen oder Formkissen aus Schaumstoffen eingesetzt werden. Als Materialien für den Kissenbezug kommen Baumwolle, Wolle, Nessel, Leinen, Seide in Betracht. Das Material des Kissenbezuges kann aus mehreren Materialbahnen gesteppt sein, auch unter Zwischenlage von Vliesmaterial. Auch kommt ein Webflorgewebe in Betracht.

Nach einer weiteren Ausgestaltung der Erfindung ist vorgesehen, daß die Bauchigkeit je nach Kissengröße zwischen 6 und 20 cm beträgt. Dabei ist zugleich in weiterer Ausgestaltung vorgesehen, daß das Abrollgebilde 3 bis 15 cm vom nackenseitigen Kissenrand entfernt beginnt. Durch den Abstand zwischen nackenseitigem Kissenrand und dem Ansatz des Abrollgebildes wird sichergestellt, daß die Durchblutung des Kopfes nicht unterbrochen wird. Eine Bauchigkeit zwischen 6 und 20 cm hat sich bei der Anwendung eines auffedernden nachgiebigen Kunststoffmaterials als ausreichend erwiesen, um den Kopf seitlich wegzurollen.

Nach einer weiteren Ausgestaltung der Erfindung ist vorgesehen, daß der Kunststoffschaum eine höhere Stauchhärte und eine Raumdichte von mindestens 30 bis 60 kg/dm³ hat. Ein solches Material hat die genügenden Auffederungseigenschaften bei ausreichender Eindrückbarkeit.

Nach einer weiteren Ausgestaltung der Erfindung ist vorgesehen, daß das Formkissen in den beiderseits des Abrollgebildes gelegenen Seitenrändern Einmuldungen für eine freie Nasenatmung aufweist. Durch das seitliche Abrollen des Kopfes auf dem Formkissen wird die Nase auch bei vom Kissen geschlossenen Mund freiliegen und damit die Atemfreiheit gewährleistet sein.

Nach einer weiteren Ausgestaltung der Erfindung ist vorgesehen, daß das Abrollgebilde mittels einer von der Außenseite des Kissens her bedienbaren Aufblasvorrichtung aufblasbar ist. Die einfachste Form einer solchen Aufblasvorrichtung ist ein von Hand betätigter Blaseball.

Nach einer weiteren Ausgestaltung der Erfindung ist vorgesehen, daß die Aufblasvorrichtung eine elektrische Pumpe ist, mit der nach Maßgabe eines im Schlafbereich vorgesehenen Geräusch- oder Drucksensors das Abrollgebilde aufblasbar ist. Eine solche Pumpe kann außerhalb des Bettes aufgestellt werden und das Kissen generell oder nur beim Schnarchen und während des Schlafes von einem normalen Schlafkissen in ein Antischnarchkissen umwandeln. Der Drucksensor wird dabei im Kissen im Bereich des Abrollgebildes angeordnet werden, während der Geräuschsensor im Kissen oder außerhalb desselben im Nahbereich des Schnarchens angeordnet wird.

Nach einer weiteren Ausgestaltung der Erfindung ist vorgesehen, daß die Aufblasvorrichtung das Abrollgebilde nach Maßgabe eines Zeitschaltgliedes im ständigen Wechsel aufbläst und sich wieder entleeren läßt. Ein solches Zeitschaltglied kann durch Aufblasen und Entleeren des Abrollgebildes den Kopf des Schläfers in Bewegung halten und ihn allein schon dadurch am Schnarchen hindern, weil der Schläfer nämlich nur schnarcht, wenn der Kopf zur Ruhe kommt.

Nach einer weiteren Ausgestaltung der Erfindung ist vorgesehen, daß beiderseits des Abrollgebildes an den Kissenseitenrändern in den Bereichen, in denen die Mundpartie des Schläfers zu liegen kommt, Mundpartieerhöhungen vorgesehen sind, die den Mund des Schläfers zudrücken. Durch dieses Mundzudrücken wird das Schnarchen unterdrückt oder das im Umfeld hörbare Schnarchgeräusch gedämpft.

Nach einer weiteren Ausgestaltung der Erfindung ist vorgesehen, daß die Mundpartieerhöhungen mittels Aufblaskörpern bildbar sind, welche mit dem Abrollgebilde mittels Luftleitungen mit dem Inneren des Abrollgebildes in Verbindung stehen. Die Luftpumpe kann damit gleichzeitig das Abrollgebilde und die Aufblaskörper der Mundpartieerhöhungen aufblasen.

Die Erfindung wird anhand der Zeichnung näher erläutert.
Es zeigen:
Fig. 1 ein Kissen zum Reduzieren des Schnarchens mit einem bauchig gewölbten und gestreckten, auffedernden Abrollgebilde in Kissenmitte und mit entsprechend wirkenden Mundpartieerhöhungen an den Seitenrändern,
Fig. 2 das Kissen nach Fig. 1 mit inaktiviertem Abrollgebilde und inaktivierten Mundpartieerhöhungen, sodaß das Formkissen den Anschein erweckt, als seien die Antischnarchmittel in Form des Abrollgebildes und der Mundpartieerhöhungen nicht vorhanden,
Fig. 3 das Kissen mit Abrollgebilde und Mundpartieerhöhungen aus auffedernden Materialien, die ihre Wirkung ständig ausüben,
Fig. 4 das Kissen nach Fig. 1 und 2, bei dem das Abrollgebilde und die Mundpartieerhöhungen aus aufblasbaren Schlauchteilen bestehen und damit entsprechend Fig.2 ohne Aufbiasen wirkungslos sind, mit einer angedeuteten gesteuerten Aufblasvorrichtung,
Fig. 5 ein entsprechend den Fig. 1, 3 und 4 ausgestattetes Kissen, auf dem der Kopf eines Schläfers ruht mit dem in Wirkung befindlichen Abrollgebilde, das den Kopf des Schläfers zur Seite gerollt hat, und einer Mundpartieerhöhung, die gegen den Mund des Schläfers drückt,
Fig. 6 das Kissen nach Fig. 1 oder 3 mit einem sich am Boden eines Kissens bzw.einer Matratze abstützenden, verbreiterten Fußteil in Seitenansicht,
Fig. 7 das Abrollgebilde nach den Fig.1 und 3 im Schnitt längs der Linie VII-VII nach Fig.6,
Fig. 8 einen Kissenbezug mit einem Abrollgebilde und Mundpartieerhöhungen, die aufblasbar sind, mit einer Aufblasvorrichtung nach einer der Figuren 1 und 4,
Fig. 9 die Ausführungsform des Kissenbezuges nach Fig.8 im Schnitt IX-IX nach Fig.8.

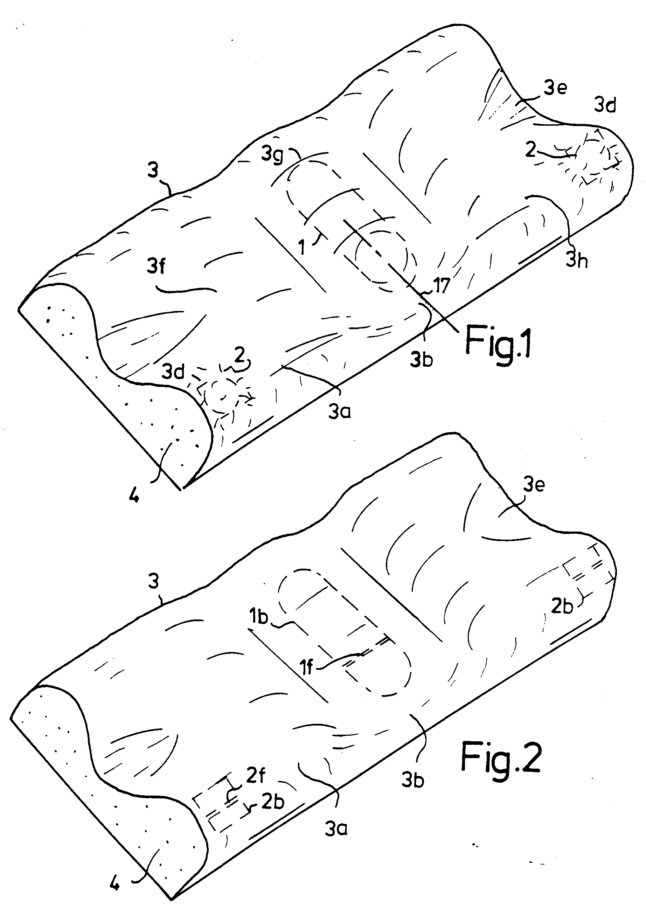

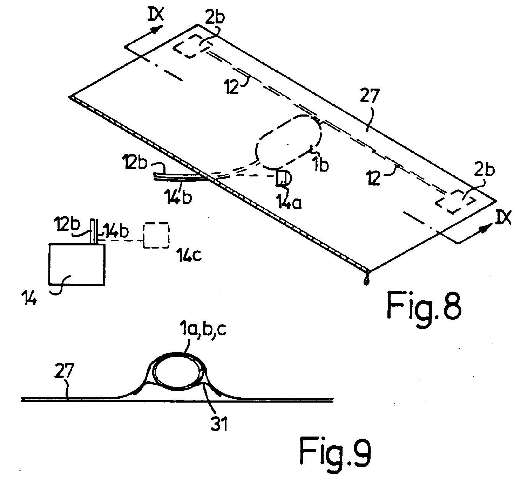

In Fig.1 sind ein Abrollgebilde 1 und Mundpartieerhöhungen 2 zum Reduzieren des Schnarchens angedeutet, die in Verbindung mit einem Kissen, vorzugsweise einem Formkissen 3, zum Einsatz kommen. Das Formkissen 3 ist an seiner Oberseite profiliert; es weist quer zur Körperlängsrichtung 17 eines Schläfers eine Nackenwelle 3a auf, durch die sich in Körperlängsrichtung 17 und damit quer zu ihr eine Nackenmulde 3b erstreckt. Parallel zur Nackenwelle 3a verläuft anschließend an diese eine Schädelmulde 3f. Das Abrollgebilde 1 erstreckt sich ebenfalls in Körperlängsrichtung 17 und schließt sich fluchtend an die Nackenmulde 3b an unter Ausbildung einer Rollwelle 3g. Die Mundpartieerhöhungen 2 befinden sich in der Nackenwelle 3a nahe der Kissenseitenränder 4. Das Formkissen 3 kann auch von anderer Gestalt sein. Wichtig sind die Lage des Abrollgebildes 1, sowie der Mundpartieerhöhungen 2 und deren Aufbau. Diese Teile können in das Formkissen eingebettet oder auf dieses aufgeklebt sein; sie müssen aber auch nicht unbedingt Bestandteile des Formkissens 3 sein; sie können auch in einen Kissenbezug integriert sein, wie es in Fig. 8 dargestellt und dazu beschrieben ist. Während des Schlafens muß eine Zuordnung zwischen dem Formkissen oder Kissenbezug mit dem Abrollgebilde 1 und den Mundpartieerhöhungen 2 bestehen, sofern beide Teile zum Einsatz kommen. Das Abrollgebilde 1 kann auf jedem Fall auch ohne die Mundpartieerhöhungen 2 eingesetzt werden.

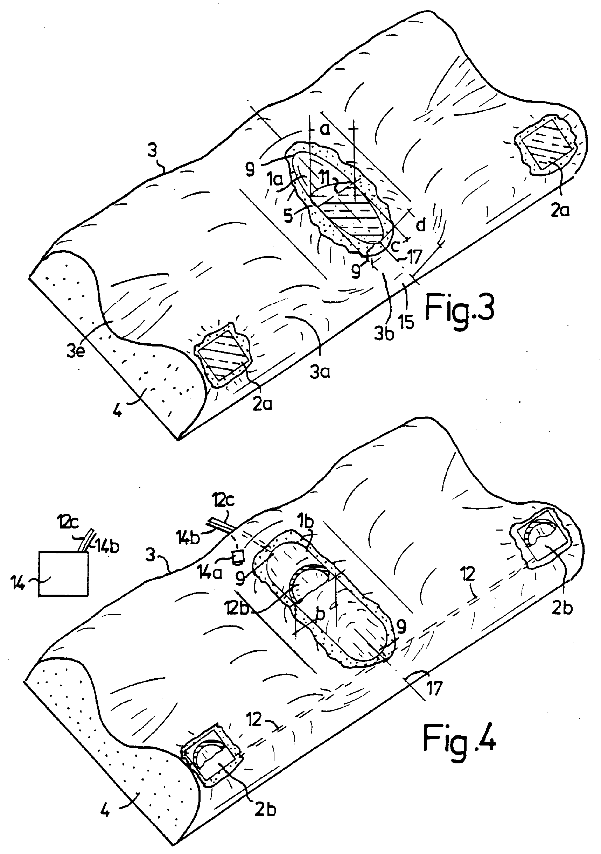

In Fig. 2 ist dargestellt, daß das Formkissen 3 auch als ein einfaches Formkissen ohne die Antischnarchteile, wie das Abrollgebilde 1 und gegebenenfalls die Mundparteierhöhungen 2, einsetzbar ist. Es gibt dann keinen in Fig. 1 dargestellten, in Fig. 2 fehlenden, vom Abrollgebilde 1 aufgewölbten Abrollkissenbereich und keine von den Mundpartieerhöhungen 2 aufgewölbte Mundschließbereiche 3d. Das Abrollgebilde 1 und die Mundpartieerhöhungen 2 sind in diesem Fall, wie in Fig.2 durch gestrichelte Parallelstriche 1f und 2f angedeutet ist, flach; sie werden erst später auf die zu Fig. 4 und 8 beschriebene Weise gebildet.

Die einfachste Art des Abrollgebildes 1 besteht, wie Fig. 3 zeigt, aus einem wulstartigen, bauchig gewölbten und langgestreckten Abrollgebilde 1a, das von seinem dem Körper des Schläfers zugewandten Ende 9 her nach oben ansteigend und abgerundet ausgebildet ist. Dieses Abrollgebilde 1a weist in seinen Grundzügen die Form eines Rugbyballes auf. Wie der Querschnitt im Bereich 5 nach Fig.3 zeigt, ist das Abrollgebilde 1a etwa oval gehalten. Dieses Abrollgebilde 1a kann entweder aus Hartschaum bestehen, der nachgiebige, auffedernde Eigenschaften aufweist mit einer höheren Stauchhärte und mit einer Schaumdichte von mindestens 30 bis etwa 60 kg/dm3, oder aus flexiblem Latexgummi. Das Abrollgebilde 1a aus Schaumstoff kann aber auch eine Walzenform mit abgerundeten Längsenden aufweisen, wie sie zum aufblasbaren Abrollgebilde 1b dargestellt ist. Zusammen mit dem Abrollgebilde 1a können in der Nackenwelle 3a im Bereich des Mundschließbereiches 3c nahe der Kissenränder 4 Mundschließaufhöhungen in Form von Flauschpolstern 2a vorgesehen sein.

Wie Fig.4 zeigt, besteht bei einer anderen Ausführungsform das Abrollgebilde 1b aus einem aufblasbaren Schlauch, dessen Querschnitt im Bereich 7 etwa rund gehalten ist. Der aufblasbare Schlauch kann beispielsweise aus längs ihrer Ränder verschweißten Folien bestehen. Beide Möglichkeiten, rund oder oval, sind aber sowohl bei den Ausführungsformen nach Fig.3 als auch bei der nach Fig.4 möglich. Die Abrollgebilde 1a und 1b nach den Fig. 3 und 4 sind an ihren Längsenden 9 im wesentlichen abgeflacht, abgerundet zulaufend ausgebildet und werden zur Mitte 11 hin bauchig. Die Dimensionierung ist dabei so gewählt, daß die Bauchigkeit zwischen 6 und 20 cm gewählt ist. Diese Bauchigkeit ist durch die Linie a angegeben. Bei einer runden Querschnittsausbildung nach.Fig.4 beträgt die Höhe b in der Mitte 11 ebenfalls 6 bis 20 cm; bei einer ovalen Ausführung nach Fig.3 ist die Höhe c kleiner als die Breite.

Auch die Mundpartieerhöhungen 2b nach Fig. 4 sind aufblasbar ausgebildet; sie bestehen aus längs ihrer Ränder miteinander verschweißten elastischen Folien von vorzugsweise rechteckigem Querschnitt. Beide Mundpartieerhöhungen 2b sind über Schlauchleitungen 12 mit dem Inneren 12b des Abrollgebildes 1b verbunden. Das Abrollgebilde 1b ist wiederum über eine Schlauchleitung 12c mit einer nicht dargestellten Aufblasblase oder einer elektrischen Pumpe 14 verbunden. Das Abrollgebilde 1b und die Mundpartieerhöhungen 2b sind damit bei Bedarf gleichzeitig aufblasbar. Dies hat den Vorteil, daß die Antischnarchteile (Abrollgebilde 1b und Mundpartieerhöhungen 2b) nur bei Bedarf aufgeblasen werden müssen.

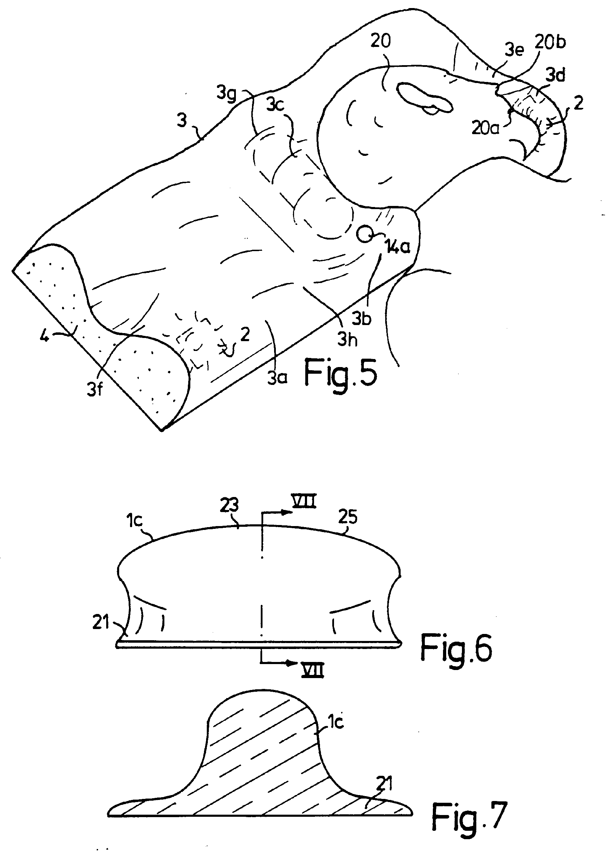

Zur Erhöhung des Komforts ist im Umfeld des Abrollgebildes 1b, also im Kissen oder außerhalb des Kissens, ein Geräuschsensor 14a vorgesehen, der über eine Kabelverbindung 14b mit einer nicht dargestellten Schaltvorrichtung der Pumpe 14 in Verbindung steht und diese ansteuert. Der Sinn dieser Anordnung ist darin zu sehen, daß sich der Schläfer zunächst bei entleertem Abrollgebilde 1b und gleichzeitig entleerten Mundpartieerhöhungen 2b bequem niederlegen kann. Beginnt der Schläfer zu schnarchen, dann meldet dies der Geräuschsensor 14a der Schaltvorrichtung der Pumpe und gibt dieser den Befehl, aufzupumpen. Nun füllen und wölben sich das Abrollgebilde 1b und die Mundpartieerhöhungen auf. Der Schläfer wendet, da das Abrollgebilde 1b gegen seinen Hinterkopf drängt, seinen Kopf zur Seite. Bald folgt dem Kopf auch unbewußt der ganze Körper. Die Mundpartieerhöhung 2b schließt ihm den Mund oder dämpft das Schnarchgeräusch (Fig.5).

An die Stelle des Geräuschsensors 14a kann auch ein Drucksensor 14a treten. Dieser Drucksensor 14a muß allerdings im Kissen angeordnet sein, da er durch den Kopf des Schläfers betätigt werden soll. Legt der Schläfer seinen Kopf auf das Kissen, dann veranlaßt der Drucksensor 14a das Aufblasen des Abrollgebildes 1b und, soweit vorhanden, der Mundpartie-Erhöhungen 2b.

Anstelle der Geräusch- oder Drucksensoren 14a kann auch ein Zeitschaltglied 14c eingesetzt werden. Dieses Zeitschaltglied muß bedienerfreundlich angeordnet sein. Im übrigen bestehen hinsichtlich der Anordnung keine Beschränkungen. Dieses Zeitschaltglied sorgt dafür, daß das Abrollgebilde 1b und, soweit vorhanden, auch die Mundpartie-Erhöhungen 2b im ständigen Wechsel aufgeblasen und wieder entleert werden. Damit bleibt der Kopf des Schläfers in ständiger Bewegung, und der Schläfer hat nicht die Gelegenheit, mit dem Schnarchen zu beginnen.

Für eine gute Wirkung des Abrollgebildes ist eine bestimmte Zuordnung zwischen dem Kissen und dem Rollgebilde erforderlich. Um die Durchblutung im Nacken nicht zu stören, ist das Abrollgebilde 1a,b so angeordnet, daß sein zur Nackenauflage 13 hingewendetes Ende 9 von der Vorderkante 15 des Formkissens im Nackenbereich einen Abstand d von 3 bis 15 cm aufweist. Das Abrollgebilde 1a,1b erstreckt sich dann mit seiner Mittellinie in der Körperlängsrichtung 17 des Schläfers.

Fig. 5 zeigt das Formkissen 3 mit wirksamem Abrollgebilde 1 und wirksamen Mundpartieerhöhungen 2. Man erkennt, wie der Kopf 20 des Schläfers von dem Abrollgebilde 1 zur Seite gewendet wurde und sein Mund 20a von.der rechten Mundpartieerhöhung 2 zugedrückt wird, während selne Nase 20b in der Nasenausnehmung 3e freiliegt. Während also der Mund beim seitlichen Abrollen des Kopfes von selbst geschlossen wird, bleibt die Nasenatmung unbehindert frei. Die Nasenausnehmungen 3e können natürlich auch von der Seite her in die Kissenseitenränder 4 eingeschnitten sein.

Fig. 6 und 7 zeigen ein Abrollgebilde 1c mit einer fußförmigen Verbreiterung 21. Diese fußförmige Verbreiterung 21 wird insbesondere dann eingesetzt werden, wenn das Kissen 3 mit einem sehr nachgiebigen Material wie Federn aufgefüllt ist. Die fußförmige Verbreiterung 21 ist so breit gehalten, daß der abrollende Kopf des Schläfers das Abrollgebilde 1c nicht beiseite kippen kann.

Die Abrollgebilde 1a, 1b können anstelle eines Einbaues auch an der Oberseite 3h des Formkissens 3 angebracht sein, beispielsweise durch Aufkleben oder Befestigen mittels eines Klettverschlusses oder eines aufgehefteten Vliesbandes.

Eine günstige Art der Anbringung des Abrollgebildes 1 und der Mundpartieerhöhungen 2 ist die Unterbringung in einem Kopfkissenbezug 27. Hier kann die Anbringung beispielsweise durch das Unterlegen eines entfernbaren Stoffstreifens 31 oder eines Vlieses erfolgen, das mittels eines Klettverschlusses an der Bezuginnenseite festklettbar ist. Ebenso gut können der Stoffstreifen 31 oder das Vlies in den Kopfkissenbezug 27 eingenäht sein. In jedem Fall bilden Stoffstreifen oder Vliesstreifen dabei eine Art Einschiebetunnel. Ein Ausführungsbeispiel dazu zeigt der Schnitt IX/IX nach Fig.8 in Fig.9. Auch bei dem Kissenbezug 27 ist ein Einsatz des aufblasbaren Abrollgebildes 1b und der aufblasbaren Mundpartieerhöhungen 2b möglich, die über die Leitungen 12 miteinander verbunden und nach Maßgabe des Geräuschsensors 14b von der Pumpvorrichtung 14 aufblasbar sind. Auch die Mundpartieerhöhungen 2b, die Leitungen 12, 12c und der Geräuschsensor 14a können mittels Vliesstreifen odgl. abnehmbar am Kissenbezug angeordnet sein.

## Patentansprüche

1. Vorrichtung zum Reduzieren des Schnarchens eines Schläfers, dessen Kopf während des Schlafens auf einem Kissen ruht, das ein Abrollgebilde (1a,1b,1c) aufweist, das sich im Kissenbereich in Verlängerung der Längsmittellinie (17) des Körpers des Schläfers erstreckt und diesen zum seitlichen Abrollen zwingt, dadurch gekennzeichnet, daß das wulstartig ausgebildete Abrollgebilde (1a,1b,1c) von seinem dem Körper zugewandten Ende (9) her und zum oben gelegenen Randbereich (23) hin abgerundet ausgebildet ist und aus einem auffedernd nachgiebigen Material besteht.

2. Vorrichtung nach Anspruch 1, dadurch gekennzeichnet, daß das Abrollgebilde (1a,1c) aus einem geformtem Kunststoffschaum oder Gummiwerkstoff, wie Latexgummi, besteht.

3. Vorrichtung nach Anspruch 1, dadurch gekennzeichnet, daß das Abrollgebilde (1b) ein luftgefüllter Aufblaskörper ist.

4. Vorrichtung nach einem der Ansprüche 1 bis 3, dadurch gekennzeichnet, daß das Abrollgebilde (1a,1b,1c) von beiden Langsenden (9) her zur Mitte (11) hin rund oder oval bauchig breiter werdend ausgebildet ist.

5. Vorrichtung nach Anspruch 1, dadurch gekennzeichnet, daß das Abrollgebilde (1a,1b,1c) an dem, seinem oben gelegenen Bereich (23) gegenüberliegenden Randbereich mit einer fußförmigen Verbreiterung (21) versehen ist.

6. Vorrichtung nach Anspruch 1, dadurch sekennzeichnet, daß das Abrollgebilde (1a,1b,1c) in oder an dem Formkissen (3) angeordnet ist.

7. Vorrichtung nach Anspruch 1, dadurch gekennzeichnet, daß das Abrollgebilde (1a,1b,1c) auswechselbar an einem Kissenbezug (27) angeordnet ist.

8. Vorrichtung nach Anspruch 4, dadurch gekennzeichnet, daß die Bauchigkeit zwischen 6 und 20 cm beträgt.

9. Vorrichtung nach Anspruch 1, dadurch gekennzeichnet, daß das Abrollgebilde (1a,1b,1c) 3 bis 15 cm vom nackenseitigen Kissenrand entfernt beginnt.

10. Vorrichtung nach Anspruch 2, dadurch gekennzeichnet, daß der Kunststoffschaum eine höhere Stauchhärte und eine Raumdichte von mindestens 30 bis 60 Kg/dm3 aufweist.

11. Vorrichtung nach Anspruch 7, dadurch gekennzeichnet, daß das Formkissen (3) in den beiderseits des Abrollgebildes (1a,1b,1c) gelegenen Seitenrändern Einmuldungen (3e) für eine freie Nasenatmung aufweist.

12. Vorrichtung nach Anspruch 3, dadurch gekennzeichnet, daß das Abrollgebilde (1b) mittels einer von der Außenseite des Kissens her bedienbaren Aufblasvorrichtung (14) aufblasbar ist.

13. Vorrichtung nach Anspruch 12, dadurch gekennzeichnet, daß die Aufblasvorrichtung eine elektrische Pumpe (14) ist, mit der nach Maßgabe eines im Schlafbereich vorgesehenen Geräusch- oder Drucksensors (14a) das Abrollgebilde (1b) aufblasbar ist.

14. Vorrichtung nach Anspruch 12, dadurch gekennzeichnet, daß die Aufblasvorrichtung (14) das Abrollgebilde (1b) nach Maßgabe eines Zeitschaltgliedes (14c) im ständigen Wechsel aufbläst und sich wieder entleeren läßt.

15. Vorrichtung nach einem der Ansprüche 1 bis 14, dadurch gekennzeichnet, daß beiderseits des Abrollgebildes (1a,1b,1c) an den Kissenseitenrändern (4) in den Bereichen, in denen die Mundpartie des Schläfers zu liegen kommt, Mundpartieerhöhungen (2) vorgesehen sind, die den Mund des Schläfers zudrücken oder das Schnarchgeräusch dämpfen.

16. Vorrichtung nach Anspruch 15, dadurch gekennzeichnet, daß die Mundpartie-Erhöhungen aus auffedernd nachgiebigem Material bestehen.

17. Vorrichtung nach Anspruch 15, dadurch gekennzeichnet, daß die Mundpartieerhöhungen (2) mittels Aufblaskörpern (2b) bildbar sind, welche mit dem aufblasbaren Abrollgebilde (2b) mittels Luftleitungen (12) mit dem Inneren (12b) des Abrollgebildes (1b) in Verbindung stehen.

## Claims

1. A device for reducing snoring of a sleeper whose head rests on a pillow during sleep, said pillow including a rolling structure (1a,1b,1c) which extends in the pillow area as an extension of the longitudinal centre line (17) of the sleeper's body and forces the head to roll sideways, characterized in that the bulge-like rolling structure (1a,1b,1c) is configured to be rounded from its end (9) facing the body and towards the topmost edge portion (23) and is made of an elastically expanding compliant material.

2. The device as claimed in claim 1, characterized in that the rolling structure (1a,1c) consists of a shaped foamed plastic or rubber material such as latex rubber.

3. The device as claimed in claim 1, characterized in that the rolling structure (1b) is an air-filled inflatable body.

4. The device as claimed in any one of the claims 1 to 3, characterized in that the rolling structure (1a,1b,1c) is configured to have a round or oval bulge of increasing width from both longitudinal ends (9) towards the middle (11).

5. The device as claimed in claim 1, characterized in that the rolling structure (1a,1b,1c) is provided with a foot-like enlargement (21) on its edge portion which is opposite to its topmost edge portion (23).

6. The device as claimed in claim 1, characterized in that the rolling structure (1a,1b,1c) is arranged in or on the shaped pillow (3).

7. The device as claimed in claim 1, characterized in that the rolling structure (1a,1b,1c) is exchangeably arranged on a pillow case (27).

8. The device as claimed in claim 4, characterized in that the bulginess is between 6 and 20 cm.

9. The device as claimed in claim 1, characterized in that the rolling structure (1a,1b,1c) commences at a distance of from 3 to 15 cm from the neck-side pillow edge.

10. The device as claimed in claim 2, characterized in that the foamed plastic has a higher resistance to compression and a bulk density of at least 30 to 60 kg/dm³.

11. The device as claimed in claim 7, characterized in that the shaped pillow (3) is provided with cavities (3e) in the side edges on either side of the rolling structure (1a,1b,1c) for unobstructed nasal respiration.

12. The device as claimed in claim 3, characterized in that the rolling structure (1b) is adapted to be inflated by means of an inflation device (14) operated from outside of the pillow.

13. The device as claimed in claim 12, characterized in that the inflation device is an electric pump (14) by means of which the rolling structure (1b) can be inflated in response to a sound or pressure sensor (14a) disposed in the sleeping area.

14. The device as claimed in claim 12, characterized in that the inflation device (14) causes the rolling structure (1b) to be continually alternatingly inflated and deflated in response to a timing circuit (14c).

15. The device as claimed in any one of the claims 1 to 14, characterized in that raised mouth portions (2) are provided on either side of the rolling structure (1a,1b,1c) on the side edges (4) of the pillow in those areas where the sleeper's mouth will rest, said raised mouth portions urging the sleeper's mouth closed or damping the snoring sound.

16. The device as claimed in claim 15, characterized in that the raised mouth portions are made of elastically expanding compliant material.

17. The device as claimed in claim 15, characterized in that the raised mouth portions (2) may be formed by inflatable bodies (2b) which are communicated via air conduits (12) to the interior (12b) of the rolling structure (1b).

## Revendications

1. Dispositif pour réduire le ronflement d'un dormeur, dont la tête repose sur un oreiller durant le sommeil, qui présente une conformation de roulement (1a, 1b, 1c) qui s'étend dans le domaine de l'oreiller dans le prolongement de la ligne médiane longitudinale (17) du corps du dormeur et qui contraint celui-ci au roulement vers le côté, caractérisé en ce que la conformation de roulement (1a, 1b, 1c) se présentant sous la forme d'un renflement est réalisée arrondie du côté de son extrémité (9) tournée vers le corps et en direction de la zone de bord (23) située en haut et est constituée d'un matériau élastique en détente.

2. Dispositif selon la revendication 1, caractérisé en ce que la conformation de roulement (1a, 1c) est constituée d'une mousse synthétique ou d'une matière première caoutchouteuse moulée, telle que de la gomme de latex.

3. Dispositif selon la revendication 1, caractérisé en ce que la conformation de roulement (1b) est un corps gonflable rempli d'air.

4. Dispositif selon l'une des revendications 1 à 3, caractérisé en ce que la conformation de roulement (1a, 1b, 1c) est réalisée de façon à devenir plus large du côté des deux extrémités longitudinales (9) en direction du milieu (11) suivant une forme convexe ronde ou ovale.

5. Dispositif selon la revendication 1, caractérisé en ce que la conformation de roulement (1a, 1b, 1c) est pourvue d'un élargissement en forme de pied à la zone de bord opposée à sa zone (23) située en haut.

6. Dispositif selon la revendication 1, caractérisé en ce que la conformation de roulement (1a, 1b, 1c) est disposée dans ou sur l'oreiller moulé (3).

7. Dispositif selon la revendication 1, caractérisé en ce que la conformation de roulement (1a, 1b, 1c) est disposée amovible sur une taie d'oreiller (27).

8. Dispositif selon la revendication 4, caractérisé en ce que la convexité est comprise entre 6 et 20 cm.

9. Dispositif selon la revendication 1, caractérisé en ce que la conformation de roulement (1a, 1b, 1c) débute à une distance de 3 à 15 cm du bord de l'oreiller situé du côté de la nuque.

10. Dispositif selon la revendication 2, caractérisé en ce que la mousse synthétique présente une dureté à la pression supérieure et une densité en volume d'au moins 30 à 60 kg/dm³.

11. Dispositif selon la revendication 7, caractérisé en ce que l'oreiller moulé (3) présente des enfoncements vers l'intérieur (3e), pour une respiration nasale libre, dans les bordures latérales situées de part et d'autre de la conformation de roulement (1a, 1b, 1c).

12. Dispositif selon la revendication 3, caractérisé en ce que la conformation de roulement (1b) est gonflable au moyen d'un dispositif de gonflage (14) qui peut être commandé par le côté extérieur de l'oreiller.

13. Dispositif selon la revendication 12, caractérisé en ce que le dispositif de gonflage est une pompe électrique (14), avec laquelle la conformation de roulement (1b) est gonflable selon une indication d'un capteur de pression ou de bruit (14a) prévu dans la zone de sommeil.

14. Dispositif selon la revendication 12, caractérisé en ce que le dispositif de gonflage (14) gonfle et puis laisse se vider la conformation de roulement (1b) suivant une indication d'un élément à interrupteur à minuterie (14c) en variation constante.

15. Dispositif selon l'une des revendications 1 à 14, caractérisé en ce que de part et d'autre de la conformation de roulement (1a, 1b, 1c), aux bords latéraux de l'oreiller (4), dans les zones dans lesquelles la partie buccale du dormeur vient à se reposer, il est prévu des élévations de partie buccale (2) qui ferment la bouche du dormeur ou atténuent le bruit de ronflement.

16. Dispositif selon la revendication 15, caractérisé en ce que les élévations de partie buccale sont constituées d'un matériau élastique en détente.

17. Dispositif selon la revendication 15, caractérisé en ce que les élévations de partie buccale (2) sont réalisables au moyen de corps gonflables (2b), qui par la conformation de roulement (2b) gonflable sont en liaison avec l'intérieur (12b) de la conformation de roulement (1b) au moyen de conduites d'air (12).
